# EUROPEAN PATENT APPLICATION

(11) **EP 4 434 438 A1**
(43) Date of publication of application: **25.09.2024**
(21) Application number: 23170077.4
(22) Date of filing: 26.04.2023
(51) Int. Cl.: A61B 3/00

(54) **A METHOD FOR PREDICTING THE ANTERIOR CHAMBER ANGLE AND AN APPARATUS THEREOF**

(30) Priority: 20.03.2023 KR 20230036118
(71) Applicant: Visuworks Inc., Seoul 00000 (KR)
(72) Inventor: RYU, Ik Hee, 00000 Seoul (KR); KIM, Jin Kuk, 00000 Seoul (KR); YOO, Tae Keun, 00000 Seoul (KR)
(74) Representative: Isarpatent

(57) **Abstract**

A method of predicting the anterior chamber angle of the eye of a surgical candidate for lens implantation according to an embodiment of the present invention includes obtaining input data including a lens size, obtaining a predicted postoperative anterior chamber angle based on a learning model using the input data. The learning model may be trained based on the lens size and the measured postoperative anterior chamber angle of a plurality of patients who have undergone the lens implantation in the past.

## Description

### CROSS-REFERENCE TO RELATED APPLICATION(S)

This application is based on and claims priority under 35 U.S.C. § 119 to Korean Patent Application No. 10-2023-0036118, filed on March 20, 2023, in the Korean Intellectual Property Office, the disclosure of which is incorporated by reference herein its entirety.

### BACKGROUND

### 1. Field

The present invention relates to a method for predicting the anterior chamber angle and an apparatus thereof.

### 2. Description of Related Art

Lens implantation refers to a surgery of inserting an artificial lens between iris and a crystalline lens of the eye and is effective in case of excessive myopia or thin cornea. In lens implantation, it is especially important to choose a proper lens because problems such as increased intraocular pressure or cataracts could occur if the lens is misfit. Some symptoms may require additional treatment of replacing or removing the lens. Therefore, the utmost care needs to be taken in determining a lens to accomplish a successful surgical outcome.

Recently, the field of artificial intelligence (AI) technology has been grafted into the medical field to expand its availability, and research is ongoing in the field of lens implantation to reflect user characteristics and predict the surgical results based on AI. As such, there are various attempts to elevate the success rate of lens implantation, but the research on the effect of the anterior chamber angle on the outcome of lens implantation and the related factors is lacking when compared to other studies.

### [Detailed Description of the Invention]

### [Technical Problem Sought to Be Solved]

The present invention aims to provide an apparatus for predicting the anterior chamber angle of the eye based on a learning model and a method of operating the same.

### [Means for Solving the Problem]

The method for predicting an anterior chamber angle of the eye of a surgical candidate for lens implantation according to an embodiment of the present invention may include obtaining input data including a lens size and obtaining a predicted postoperative anterior chamber angle based on a learning model using the input data.

According to one embodiment, the learning model may be trained based on the lens sizes and the measured postoperative anterior chamber angles of a plurality of patients who have undergone the lens implantation in the past.

The method of predicting the anterior chamber angle of the eye for lens implantation according to an embodiment may further include outputting the obtained lens size.

According to one embodiment, the obtained lens size may be determined based on a bolting value.

According to one embodiment, the input data may further include examination data of the surgical candidate.

According to one embodiment, the examination data may further include at least one of the pupil size, the anterior chamber depth (ACD), the anterior chamber width (ACW), the crystalline lens rise (CLR), or the preoperative anterior chamber angle.

According to one embodiment, the anterior chamber angle may be defined as a trabecular iris angle (TIA) or a scleral spur angle (SSA).

An apparatus for predicting the postoperative anterior chamber angle of the eye according to an embodiment of the present invention includes a memory for storing a learning model trained based on the lens size and the measured postoperative anterior chamber angle of a plurality of patients who have undergone the lens implantation in the past, and at least one processor electrically connected to the memory.

According to an embodiment, the processor may obtain input data including a lens size and obtain a predicted postoperative anterior chamber angle based on a learning model using the input data.

### [Advantageous Effects of the Invention]

According to the present invention, the learning model can be used to predict the outcome of lens implantation surgery effectively and accurately.

According to the present invention, side effect occurrence caused by the lens implantation surgery can be reduced by predicting the surgical outcome with the learning model.

### [Brief Description of the Drawings]

To more thoroughly understand the drawings cited in the detailed description of the present invention, a brief description of each drawing is provided as follows.
Fig. 1 illustrates an operative environment of the apparatus for predicting the anterior chamber angle based on a learning model according to an embodiment.
Fig. 2 is a block diagram illustrating a configuration of the apparatus according to an embodiment.
Fig. 3 illustrates a relationship between the devices according to an embodiment.
Fig. 4 illustrates a learning model according to an embodiment of the present invention.
Fig. 5 is a diagram explaining the anterior chamber angle according to an embodiment.
Fig. 6 illustrates a system of predicting the anterior chamber angle according to an embodiment.
Fig. 7 is a diagram of a learning flow of the electronic device according to an embodiment.
Fig. 8a is a diagram illustrating an architecture for predicting the anterior chamber angle according to an embodiment.
Fig. 8b is a diagram illustrating the method of predicting the anterior chamber angle according to an embodiment.
Fig. 8c is a diagram explaining the method of predicting the anterior chamber angle according to an embodiment.
Fig. 9 is a flowchart of the method of predicting the anterior chamber angle according to an embodiment.
Fig. 10 is a flowchart of the method of predicting the anterior chamber angle according to an embodiment.
Fig. 11 is a diagram illustrating an architecture for predicting the anterior chamber angle according to an embodiment.
Fig. 12 is a flowchart of a method of predicting the anterior chamber angle according to an embodiment.
Fig. 13 depicts a relationship between the preoperative eye measurement data and the postoperative anterior chamber angle.
Fig. 14 depicts the results of multivariate LR analysis for postoperative TIA.
Fig. 15 depicts the results of multivariate LR analysis for postoperative SSA.
Fig. 16 depicts the Shapley value of each predictor for the anterior angle prediction model according to various embodiments of the present invention.
Fig. 17 depicts the results of comparing the predicted postoperative anterior chamber angle with the actual anterior chamber angle according to various embodiments of the present invention.
Fig. 18 depicts the results of comparing the predicted postoperative anterior chamber angle with the actual anterior chamber angle according to various embodiments of the present invention.
Fig. 19 depicts the results of comparing the actual postoperative anterior chamber angle with the predicted anterior chamber angle according to various embodiments of the present invention.
Fig. 20 depicts the ROC AUC of the anterior chamber angle prediction model according to various embodiments of the present invention.
Fig. 21 depicts a change in the anterior chamber angle according to a change in the lens size.

### [Detailed Description for Practicing the Invention]

Hereinafter, various embodiments of the present invention will be described with reference to the attached drawings. The present invention is not limited to the specific embodiments and should be construed to encompass various modifications, equivalents, and/or alternatives of embodiments of the present invention. In connection with the description of the drawings, similar reference numerals may be used for similar components.

In this document, terms such as "have," "may have," "comprises," or "may include" indicate the presence of the corresponding feature (e.g., a numerical value, function, operation, or component such as a part) and does not exclude the presence of additional features.

In this document, terms such as "A or B," "at least one of A or/and B," or "one or more of A or/and B" may include any possible combination of the items listed together. For example, "A or B," "at least one of A and B," or "at least one of A or B" may indicate (1) containing at least one A, (2) containing at least one B, or (3) referring to both including at least one A and at least one B.

The terms "first" and "second" used herein are intended to identify different components and are not intended to denote any order or hierarchy of importance. Therefore, these terms do not limit the components. For instance, the first component may be referred to as the second component or vice versa without departing from the scope of this document.

The term "configured to" as used herein may be used interchangeably with, for example, "suitable for," "having the capacity to," "designed to," "adapted to," "made to," or "capable of," depending on the situation. The term "configured to" does not necessarily mean only "specifically designed to."

In this document, the terms "command," "instruction," "control information," "message," "information," "data," "packet," "data packet," "intent," and/or "signal" refer to the various forms of communication that are transmitted and received between the first electronic device(s) and the second electronic device(s), and may include an idea or a concrete electric representation (e.g., digital signs or analog physical quantities), or refer to themselves regardless of the expression. It will be apparent to one with ordinary skills in the art that the exemplary expressions listed above can be interpreted variously depending on the context in which they are used. In this document, "greater than B" means not only "greater than B," but also "equal to or greater than B." The terms used herein are only to describe a particular embodiment and may not be intended to limit the scope of the other embodiments. A singular expression may include a plural expression unless the context clearly indicates otherwise. All terms including technical and scientific terms used herein have the same meaning as commonly understood by one with ordinary skills in the art to which this invention pertains. It should be noted that the terms used in this document, including those found in commonly used dictionaries, should be interpreted within the context of the relevant art and not in an idealized or overly formal sense, unless expressly defined as such within this document. In some cases, even the terms defined herein cannot be construed to exclude the embodiments herein.

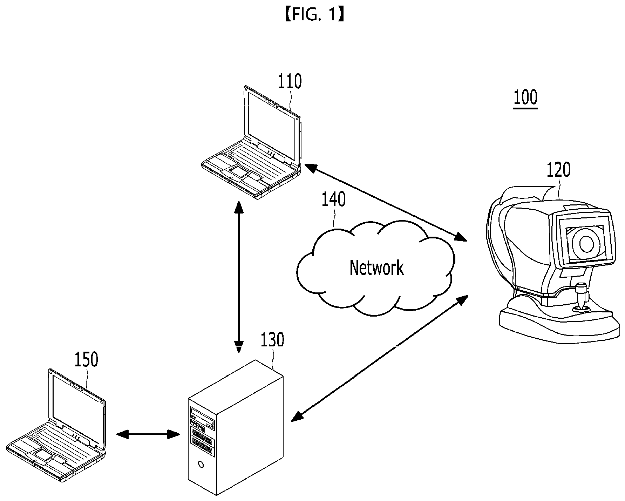

Fig. 1 illustrates an operative environment of the apparatus for predicting the anterior chamber angle based on a learning model according to an embodiment.

According to an embodiment, the operative environment 100 of the apparatus for predicting the anterior chamber angle may include at least one electronic device 110, 120, 130 and a server 140. In addition, the devices may communicate with each other via a network 150.

According to one embodiment, a first electronic device 110 may be an apparatus for performing the anterior chamber angle prediction according to various embodiments of the present invention. The first electronic device 110 may perform all or part of the operation described below, for example, as an apparatus for processing an operation based on a learning model by obtaining input data.

A second electronic device 120 may be an apparatus for measuring the state of the eye of a subject. The second electronic device 120 may perform all or part of the operation described below, for example, as an apparatus for obtaining the examination data of the subject or for processing an operation by obtaining the examination data of the subject.

A third electronic device 130 may be a training apparatus for training the anterior chamber angle prediction model. The third electronic device 130 may specifically train the anterior chamber angle prediction model based on the learning dataset.

The server 140 may be a device for storing the anterior chamber angle prediction model or performing the anterior chamber angle prediction according to various embodiments of the present invention. The server 140, for example, may store the anterior chamber angle prediction model in a database and transmit the anterior chamber angle prediction model upon request of the electronic device. Alternatively, server 140 may store the anterior chamber angle prediction model and, upon request for the electronic device, directly perform the anterior chamber angle prediction and transmit the prediction performance result to the electronic device.

According to one embodiment, the first electronic device 110 may store and/or drive the anterior chamber angle prediction model. The first electronic device 110 may store the weights applied to the trained anterior chamber angle prediction model. The first electronic device 110 may collect and/or store the data used for the anterior chamber angle prediction. For example, the first electronic device 110 may obtain the examination data from the second electronic device 120. In addition, the first electronic device 110 may obtain the anterior chamber angle prediction model from the server 140 or the third electronic device 130.

The first electronic device 110 may perform an operation based on the anterior chamber angle prediction model using the input data. The first electronic device 110 may obtain the output data through the operation based on the anterior chamber angle prediction model.

The first electronic device 110 may communicate with the second electronic device 120, the third electronic device 130, or the server 140 to receive or transmit data such as input data or a learning model.

According to an embodiment, the second electronic device 120 may be a measuring device for measuring an eye and may be an apparatus for measuring the anterior eye portion of the eye using a laser and/or high-frequency ultrasound. For example, the second electronic device 120 may include ultrasound biomicroscopy (UBM), optical coherence tomography (OCT), or the like. The second electronic device 120 may transmit the obtained examination data to the first electronic device 110 or the server 140.

The second electronic device 120 may perform all or part of the operation of the first electronic device 110, the third electronic device 130, or the server 140. For example, the second electronic device 120 may drive the anterior chamber angle prediction model to obtain a result of the anterior chamber angle prediction without providing the examination data to the first electronic device 110 or the server 140.

The third electronic device 130 may receive training data from the first electronic device 110, the second electronic device 120, or the server 140. The third electronic device 130 may transmit the anterior chamber angle prediction model generated based on the learning data to an external device, for example, the first electronic device 110 or the server 140.

The third electronic device 130 may perform all or part of the operation of the first electronic device 110, the second electronic device 120, or the server 140. For example, the third electronic device 130 may train the anterior chamber angle prediction model and drive the trained anterior chamber angle prediction model. For example, the third electronic device 130 may obtain the examination data from the second electronic device 120 and generate output data using the anterior chamber angle prediction model.

Upon request of the first electronic device 110, the server 140 may transmit the anterior chamber angle prediction model to the first electronic device 110. The server 140 may store and/or drive the anterior chamber angle prediction model. The server 140 may store weights applied to the trained anterior chamber angle prediction model. The server 140 may collect and/or store data used for the anterior chamber angle prediction.

The server 140 may perform all or part of the operation of the first electronic device 110, the second electronic device 120, or the third electronic device 130. For example, the server 140 may obtain the input data from the first electronic device 110 or the second electronic device 120, perform the anterior chamber angle prediction based on the anterior chamber angle prediction model, and transmit the outcome to the electronic device 110 or the second electronic device 120.

The server 140 may include one or more servers, e.g., a cloud of servers. For the convenience of description, various operations will be described based on a single server, but the operations provided in the specification of the present invention may be performed by one or more servers, and each different operation may be performed by the same or different servers.

According to one embodiment, network 150 may be directly or indirectly communicatively coupled with the first electronic device 110, the second electronic device 120, the third electronic device 130, and/or the server 140. The network 150 may include a wired or wireless communication network. In addition, network 150 may include a short-distance or long-distance network. For example, network 150 may include cellular networks such as LTE, LTE-A, 5G, and 6G.

The configuration of Fig. 1 is exemplary and thus is not intended to limit the scope of the present invention. The apparatus according to the present invention can operate in various environments, which may include some or all the configurations depicted in Fig. 1, as well as additional or different configurations not shown in Fig. 1.

In the following embodiments, the description will mainly be on the operation of the first electronic device 110, but some or all the operations described below may be performed by the second electronic device 120 or the server 140.

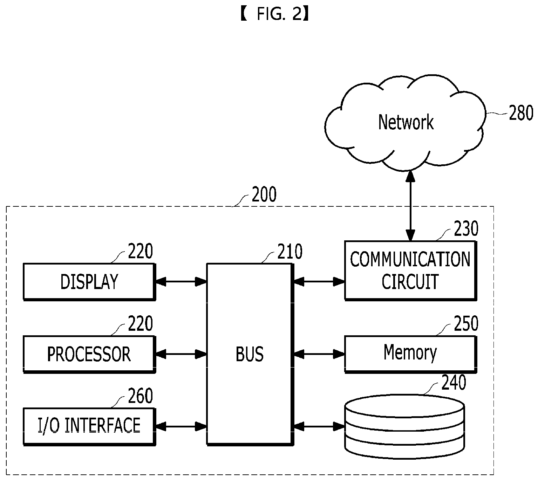

Fig. 2 is a block diagram illustrating a configuration of the device according to an embodiment.

As shown in Fig. 2, the electronic device 200 (e.g., the first electronic device 110 of Fig. 1) includes a bus 210, a display 220, a communication circuit 230, a database 240, a memory 250, an input/output (I/O) interface 260 and a processor 270. In another embodiment, device 200 may omit at least one of the components or additionally include other components.

For reference, the components 210, 220, 230, 240, 250, 260, and 270 shown in Fig. 2 as part of the electronic device 200 are provided as examples for describing a method of supporting document creation in accordance with an embodiment of the present invention. It should be noted that the electronic device 200 in accordance with this embodiment may include additional components beyond those depicted in the figure.

The bus 210 may electrically connect the components 220-270 to each other. The bus 210 may include circuitry for communication (e.g., control messages and/or data) between the components 220-270.

Display 220 may display texts, images, videos, icons, or symbols constituting various kinds of contents. Display 220 may include a touch screen and may receive a touch, a gesture, or a proximity or hovering input using an electronic pen or a portion of the user's body.

For example, display 220 may include a liquid crystal display (LCD), a light emitting diode (LED) display, an organic LED display or a microelectromechanical systems (MEMS) display, or an electronic paper display. Display 220 may be implemented as included in the electronic device 200 or may be implemented separately from the electronic device 200 but may be operatively connected to the electronic device 200.

The communication circuit 230 may establish a communication channel with the electronic device 200 and external devices. The communication circuit 230 may access network 280 through wireless or wired communication to communicate with external devices. In one embodiment, the communication circuit 230 may include circuitry for forming a wide area network connection or peer-to-peer connection, such as Wi-Fi, Bluetooth, and/or cellular communication circuitry.

The database 240 may be installed on memory 250 or may be installed on a separate storage medium. The database 240 may store all contents, details, and the like of data transmitted and received with an external device. The data stored in database 240 may be updated regularly according to a predetermined period.

According to an embodiment of the present invention, various information input from an external device may be stored in database 240. For example, database 240 may store questionnaire data such as the age and gender of the subject, the examination data of the subject, and the like.

According to various embodiments, since the data stored in database 240 is sensitive information of the subject, it may be distributed and stored in a block chain network to improve the security for using the information. When database 240 is distributed and stored in the blockchain network, the history of transmission, modification, deletion, addition, etc. of the information included in the database 240 can be more safely managed in the blockchain network.

The memory 250 may include a volatile and/or nonvolatile memory. The memory 250 may store instructions or data related to at least one other component in the electronic device 200. For example, the memory 250 may, upon execution, store instructions that cause the processor 270 to perform various operations described herein. For example, the instructions may be included in a package file of an application program.

The I/O interface 260 may serve to transmit the instructions or data input from a user or other external device to the other components of the electronic device 200. The I/O interface 260 may be implemented in hardware or software and may be used as a concept to encompass a user interface (UI) and a terminal for communication with other external devices.

Processor 270 may include at least one of a central processing unit (CPU), an application processor (AP), or a communication processor (CP). The processor 270 is electrically connected to the memory 250, the display 220, and the communication circuit 230 via the bus 210, and during operation, may execute the operation or data processing related to control and/or communication of the other components according to the instruction, program or software stored in the memory 250. Thus, the execution of the instruction, application program, or software may be construed as an operation of processor 270.

According to an embodiment of the present invention, processor 270 may perform data processing for performing the training and determining steps of the model related to the anterior chamber angle prediction described below. According to an embodiment of the present invention, the processor 270 may perform training the model related to the anterior chamber angle prediction described below and may generate the outcome of the anterior chamber angle prediction using the model related to the anterior chamber angle prediction.

Network 280 may include at least one of a telecommunications network, a computer network, the Internet, or a telephone network. Wireless communication protocols for accessing the network 280 may use, for example, at least one of the long-term evolution (LTE), LTE advanced (LTE-A), code division multiple access (CDMA), Wideband CDMA (WCDMA), universal mobile telecommunications system (UMTS), wireless broadband (WiBro), global system for mobile communications (GSM), or 5G standard communication protocols. However, they are just exemplary. Various wired and wireless communication technologies applicable in the art may be used according to the embodiment to which the present invention is applied.

According to the electronic device 200 in accordance with an embodiment of the present invention, it is possible to provide a strategy for the lens implantation which can minimize inspection for the anterior chamber angle prediction, save human, social, and economic costs, and examination time, and reduce side effects caused by the lens implantation.

Fig. 2 depicts exemplary elements included in an electronic device (e.g., electronic device 110 of Fig. 1), but some or all these elements may be included in a server (e.g., server 140 of Fig. 1).

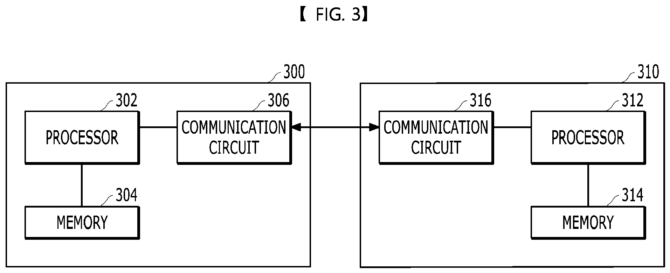

Fig. 3 depicts a relationship between devices according to an embodiment.

In view of Fig. 3, the electronic device 300 (e.g., the first electronic device 110 of Fig. 1) may interact with the external device 310 (e.g., the second electronic device 120 or server 140 of Fig. 1) for the anterior chamber angle prediction.

According to one embodiment, the electronic device 300 may include at least one of the processor 302, the memory 304, or the communication circuit 306. Processor 302 may control the communication circuit 306 to communicate with the external device 310. Communication circuit 306 may support wired or wireless communication. In addition, communication circuit 306 may support bidirectional or unidirectional communication.

According to one embodiment, the processor 300 may obtain the examination data from the external device 310 through the communication circuit 306. The electronic device 300 may obtain a learning model from the external device 310 through communication circuit 306. The processor 300 may transmit output data to the external device 310 through communication circuit 306.

According to one embodiment, external device 310 may include at least one of processor 312, memory 314, or communication circuit 316. Processor 312 may control communication circuit 316 to communicate with electronic device 310. Communication circuit 316 may support wired or wireless communication. In addition, communication circuit 316 may support bidirectional or unidirectional communication.

According to an embodiment, processor 310 may transmit the examination data to the external device 310 through the communication circuit 316. The external device 310 may transmit the learning model to the electronic device 300 through the communication circuit 316. The processor 310 may receive the output data from the external device 310 through the communication circuit 316.

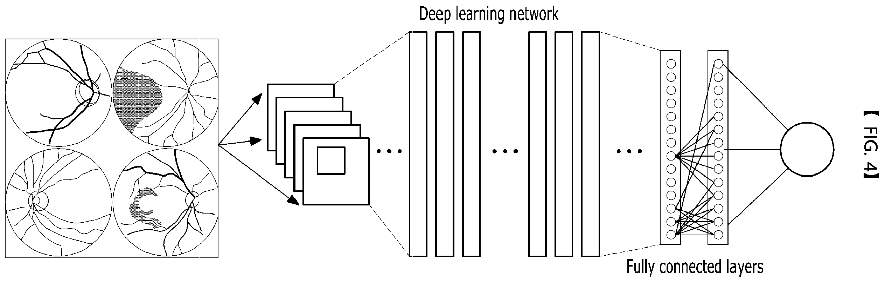

Fig. 4 illustrates a learning model according to an embodiment of the present invention.

The learning model according to an embodiment of the present invention may include a neural network for predicting the postoperative anterior chamber angle of the surgical candidate. According to an embodiment of the present invention, the neural network may consist of a set of interconnected node units. A plurality of nodes means a plurality of neurons. According to an embodiment of the present invention, nodes constituting a neural network may be connected by one or more links. In a neural network, one or more nodes connected via a link may form a relationship between an input node and an output node.

In the relationship between an input node and an output node connected through one link, the value of the data of the output node may be determined according to the data input to the input node. According to an embodiment of the present invention, the link interconnecting the input node and the output node may have a weight. The weight may be variable. In addition, the neural network according to an embodiment of the present invention may vary the weight by the user or predetermined algorithm to predict the postoperative anterior chamber angle. For example, if one or more input nodes are connected to one output node by link, the output node may determine a value of the output node based on the values input to the input nodes connected to the corresponding output node and the weight set in each link corresponding to the input nodes.

According to an embodiment of the present invention, the performance of the neural network can be determined depending on the numbers of the nodes and the links in the neural network, the associative relationship between the nodes and the links, and the weight value assigned to each of the links. For example, when there are two neural networks in which the number of the nodes and the number of the links are the same while the weight values of the links are different, the two neural networks may be recognized as being different.

A neural network may consist of a plurality of nodes, and a subset of the nodes constituting a neural network may be referred to as a layer. Some of the nodes that constitute a neural network may construct layers based on the distance they form from a particular input node. For example, a set of the plurality of nodes having a distance of "n" from a particular input node may constitute an "n"-layer. The distance from a particular input node means the number of links that must be gone through to reach the node from the particular input node. However, the definition of such a layer is merely an embodiment and thus is not limited thereto.

According to an embodiment of the present invention, the number of nodes included in the input layer may be equal to the number of nodes included in the output layer. In a neural network according to another embodiment of the present invention, the number of nodes of the input layer may be different from the number of nodes of the output layer.

A deep neural network (DNN) means a neural network that includes at least one hidden layer as well as an input layer and an output layer. The deep neural network can be used to identify latent structures in the data. The deep neural network may include convolutional neural networks, recurrent neural networks, generative adversarial networks, auto encoders, deep belief networks (DBNs), and the like. However, this is merely exemplary and is thus not limited thereto.

The neural network may be trained in at least one manner of supervised learning, unsupervised learning, semi supervised learning, reinforcement learning, and imitation learning. Training the neural network may be a process in which the neural network builds a specific model by applying the information (data) to the neural network to predict the postoperative anterior chamber angle.

The neural network may be trained based on the training data so that the error of the output can be minimized. The neural network may repeatedly receive the training data and calculate an error between the output of the neural network and target data based on the training data. To reduce the calculated error, the neural network backpropagates the error of the neural network in a direction from the output layer (or the output node) to the input layer (or the input node) of the neural network, to update the weight of each node of the neural network.

In the case of supervised learning, the neural network may use the training data (labeling data) with the correct answer value labeled in each training data. In the case of unsupervised learning, the neural network may not have the correct answer labeled in each training data. For example, the training data used for the supervised learning on data classification may be the data labeled with a category in each training data. The neural network can receive the labeling data input and calculate an error by comparing the output of the neural network with the label of the training data. As another example, for the training data, which is an input used for the unsupervised learning on data classification, the neural network may calculate an error while comparing it with the output of the neural network. The calculated error can propagate backwards in the neural network. The neural network can update the weight corresponding to the link of each node included in each layer of the neural network according to backpropagation. A variation of the connection weight of each node to be updated may be determined by the learning rate. The computation of the neural network on the input data and the backpropagation of errors can constitute a learning cycle. The learning rate can be applied differently depending on the number of iterations of the neural network's learning cycle.

In training a neural network, the training data may generally be a subset of the actual data (i.e., the eye state information to be processed using the trained neural network, etc.). There may be a case in which the errors increase for the actual data whereas the errors for the training data decrease. Overfitting refers to a phenomenon in which the training data is overtrained and the error increases for the actual data. Overfitting can contribute to an increase in errors in machine learning algorithms.

To prevent overfitting, methods can be used such as regularization to increase the training data, dropout to deactivate a part of the node in the network in the training process, and batch normalization layer.

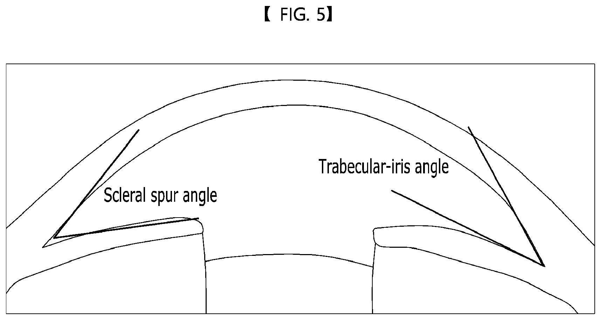

Fig. 5 is a diagram for explaining the anterior chamber angle according to various embodiments of the present invention.

Trabecular Iris Angle (TIA) or Scleral Spur Angle (SSA) can be used as the indicators to measure the anterior chamber angle (ACA).

TIA can be defined as the angle between an imaginary line that runs from a point at the Schlemm's canal along the Schwalbe line for a predetermined distance, and another imaginary line that is drawn to the Schlemm's canal from the point where the line running perpendicularly towards the iris meets the iris. The case based on the distance of 500µm is called TIA500, and the case based on the distance of 750 µm is called TIA750.

SSA can be defined as the angle between an imaginary line that runs from the scleral spur to a point along the Schwalbe line for a predetermined distance, and another imaginary line that is drawn to the scleral spur from the point where the line running perpendicularly towards the iris from the line meets the iris. The case based on the distance of 500µm is called SSA500, and the case based on the distance of 750 µm is called TIA750.

The anterior chamber angle can be measured using ocular optical tomography or the like. For example, a measuring device (e.g., the second electronic device 120 of Fig. 1) may include a device such as an optical coherence tomography (OCT) or an ultrasound biomicroscopy (UBM).

The anterior chamber angle presented in Fig. 5 is exemplary, and various indicators for measuring the anterior chamber angle of the eye may be applied to various embodiments of the present invention.

After the lens implantation surgery, the anterior chamber angle of the eye may decrease, which can have a serious side effect, such as affecting aqueous humor flows. Bolting values are actively studied in connection with successful lens implantation, but the bolting values alone may not be sufficient to deduce the successful lens implementation results. For safer and more accurate lens implantation, additional models are needed to predict the postoperative anterior chamber angle.

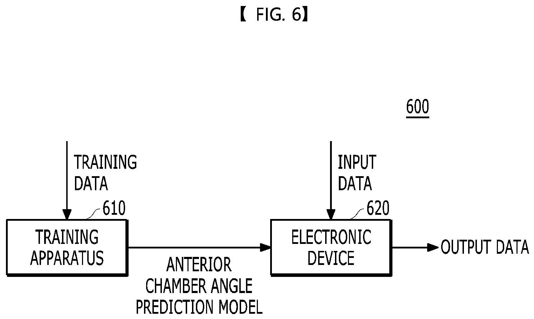

Fig. 6 conceptually depicts the anterior chamber angle prediction system according to an embodiment.

According to one embodiment, an anterior chamber angle prediction system 600 includes a training apparatus 610 (e.g., the third electronic device 130 of Fig. 1) and an electronic device 620 (e.g., the first electronic device 110 of Fig. 1 or server 140).

According to one embodiment, the training apparatus 610 may train the anterior chamber angle prediction model. Specifically, the training apparatus 610 may train the anterior angle prediction model based on the training data. The training apparatus 610 may train the anterior chamber angle prediction model in various ways such as supervised learning, unsupervised learning, reinforcement learning, or imitation learning. The training apparatus 610 may train the anterior chamber angle prediction model using the labeled data. However, the apparatus does not necessarily use the labeled data, and it may use unlabeled data.

According to one embodiment, the trained anterior chamber angle prediction model may be transmitted to the electronic device 620. The electronic device 620 may obtain and drive the anterior chamber angle prediction model trained from the training apparatus 610. The electronic device 620 may provide information to enable the user to determine the lens to be used for the surgery based on the anterior chamber angle prediction model. In addition, although the anterior chamber angle prediction model is transmitted to the electronic device 620, the anterior chamber angle prediction model may be transmitted to a server (e.g., server 140 of Fig. 1) and stored in the server.

Fig. 6 illustrates that the training of the anterior chamber angle prediction model and the utilization of the trained anterior chamber angle prediction model are performed by separate devices, i.e., in the training apparatus 610 and the electronic device 620, yet the training and utilization of the anterior chamber angle prediction model may be performed by one device.

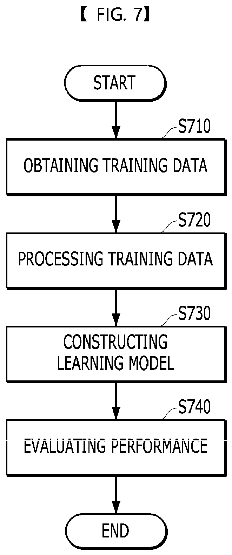

Fig. 7 is a flowchart illustrating an operation of the training apparatus according to an embodiment of the present invention. In particular, Fig. 7 decpits the operation of the training apparatus for constructing the anterior chamber angle prediction model according to an embodiment of the present invention.

In operation S710, the training apparatus (e.g., the electronic device 110 of Fig. 1, the electronic device 200 of Fig. 2, or the training apparatus 10 of Fig. 6) may collect training data. According to an embodiment of the present invention, the electronic device may receive the training data from an external device. Alternatively, the electronic device may collect the training data by itself. According to various embodiments of the present invention, the electronic device may collect the training data using data augmentation, GAN, and the like based on the anterior chamber angle stored in a database (e.g., database 240 of Fig. 2).

According to one embodiment, the training data may include at least one of the examination data, the lens information (e.g., lens size), or the postoperative anterior chamber angle data of a patient who has undergone the lens implantation in the past.

According to one embodiment, the examination data may include those obtained from the eye measurement equipment for a patient who has undergone the lens implantation in the past. In addition, the examination data may include various types of parameters for a plurality of the examination data. For example, the examination data may include at least one of the data such as SE (Spherical Equivalent), Km (mean keratometry), pupil size, IOP (intraocular pressure), WHW (White-to-white), CCT (Central Corneal Thickness), ATA (angle to angle distance), ACD (Anterior Chamber Depth), ACW (Anterior Chamber Width), CLR (Crystalline Lens Rise), lens bolting value (Lens Vault), Angle Opening Distance (AOD), Trabecular Iris Surface Area (TISA), the anterior chamber angle and the like.

The examination data may include questionnaire data. Specifically, it may include data such as age and gender of the patient.

The examination data may include the patient's medical record. The examination data may include, for example, the prescription data of the glasses worn in the past, the eyesight examination result, the refractive error (the degree of myopia, astigmatism, or hyperopia), and the like.

Information related to the lens in the training data may include the lens size inserted into the eye of the patient who has undergone the lens implantation in the past. In this case, the information related to the lens may include the lens size inserted into the the patient's eye if no side effect has occurred after the lens implantation or if the anterior chamber angle meets a predetermined standard (e.g., the anterior chamber angle is more than 20 degrees) after a predetermined period has elapsed since the lens implantation. Alternatively, the information related to the lens may include the lens size inserted into the patient's eye if a side effect occurs after the lens implantation or when the anterior chamber angle does not meet a predetermined standard (e.g., the anterior chamber angle is less than 20 degrees) after a predetermined period of time has elapsed since the lens implantation.

The postoperative anterior chamber angle data may be the anterior chamber angle data measured after the surgery of the patient. The postoperative anterior chamber angle data may include the anterior chamber angle data measured after a predetermined period of time (e.g., 6 months) has elapsed since the time of surgery.

In operation S730, the training apparatus may process a training dataset based on the training data. According to an embodiment, the training apparatus may divide each of the training data into domains, types, and clusters. The training apparatus may classify sample data into a domain, a type, and a cluster as described above based on a predetermined basis and generate a training dataset.

In operation S740, the training apparatus may construct a learning model for predicting the patient's anterior chamber angle based on the training dataset. The learning model may include a model for predicting the anterior chamber angle based on the training data. Or, the learning model may include a model for predicting the anterior chamber angle and lens size based on the training data. This learning model is hereinafter referred to as the anterior angle prediction model.

The learning model may include at least one of a plurality of learning algorithms for predicting the postoperative anterior chamber angle. For example, the algorithms include linear regression, logistic regression, K-nearest neighbors, support vector machine, decision tree, random forest, and neural networks.

The learning model may use a plurality of learning algorithms among the plurality of learning algorithms for calculating the predicted value. For example, an ensemble method may be used in the learning model, in which case, better prediction performance can be obtained than when the learning algorithms are used separately.

In operation S750, the training apparatus may evaluate the performance on the learning model. According to an embodiment of the present invention, the training apparatus may calculate an error by comparing the output data and the training data based on the learning model. The electronic device can calculate errors periodically to evaluate performance on machine learning models.

Though not depicted in the drawings, the training apparatus may update the learning model in a direction of reducing the calculated error. For example, the training apparatus obtains a result value (the output data) using a model given random weights, compares the obtained result value (the output data) with the labeling data of the training data, performs backpropagation according to the error, and optimizes the weights.

As a result of operations S710 to S750, the training apparatus may obtain parameters constituting the anterior chamber angle prediction model. The parameters may include the weight as adjusted while training the anterior chamber angle prediction model.

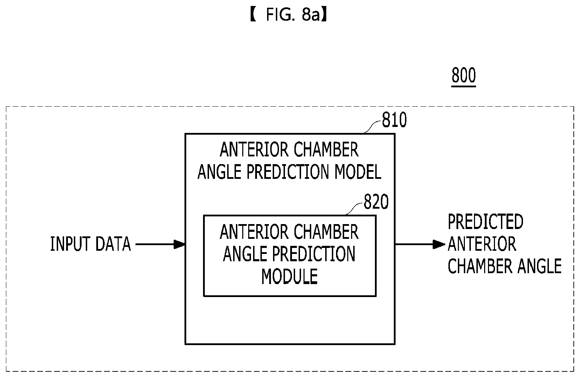

Fig. 8a illustrates an architecture for the anterior chamber angle prediction according to an embodiment.

According to one embodiment, the architecture 800 may be provided to predict the postoperative anterior chamber angle based on the input data supplied to the anterior chamber angle prediction module 810. The architecture 800 may be implemented by an electronic device (e.g., the first electronic device 110 of Fig. 1) or a processor (e.g., the processor 270 of Fig. 2) of a server (e.g., the server 140 of Fig. 1).

According to one embodiment, the anterior chamber angle prediction module 810 may include an anterior chamber angle prediction model 820. The anterior chamber angle prediction model 820 may be a learning model configured to detect the predicted postoperative anterior chamber angle of the eye based on the input data. The anterior chamber angle prediction model may have been trained based on the lens sizes and the measured postoperative anterior chamber angles of a plurality of patients who have undergone the lens implantation in the past.

According to one embodiment, the anterior chamber angle prediction model 820 may, prior to being placed on an electronic device or server, be pretrained on a different electronic device or server based on the input data or may be pretrained on the same electronic device or server.

According to one embodiment, the input data may include parameters. Depending on the types of the parameters included in the input data, the degree to which the input data affects the result value may vary.

The input data may include at least one of the examination data and the information related to the lens (e.g., a lens size) of the surgical candidate for the lens implementation.

The examination data may include the examination data obtained from the eye measurement equipment for the surgical candidate. In addition, the examination data may include various types of parameters for a plurality of the examination data. For example, the examination data may include at least one of SE, Km, pupil size, IOP, WHW, CCT, ATA, ACD, ACW, CLR, lens bolting value, AOD, TISA, the anterior chamber angle, and the like.

The examination data may include questionnaire data. Specifically, it may include data such as age and gender of the surgical candidate.

The examination data may include the medical records of the surgical candidate. The examination data may include, for example, the prescription data of the glasses worn in the past, the eyesight examination result, the refractive error (the degree of myopia, astigmatism, or hyperopia), and the like.

The examination data may include measurement data for the cornea. For example, it may include corneal form, corneal symmetry, corneal thickness measurement data, corneal structure tomography data, corneal morphology analysis data, corneal curvature, corneal endothelial cytometry data, etc.

The examination data may include measurement data for visual acuity and/or refraction. For example, it may include data on the prescription data of the glasses worn in the past, the eyesight examination result, the refractive error (the degree of myopia, astigmatism, or hyperopia), and the like.

The examination data may include measurement data for distance and the like in the eye. Specifically, it may include the pupil size, eye length, distance of space into which the lens is to be inserted, and the like.

The examination data may include data on eye disease and/or retained disease. For example, it may include the data on the presence or absence of retinal disease, glaucoma, retinal degeneration, etc., cataract, diseases of the back of the iris, and the like.

The examination data may include measurement data for the retina. For example, it may include pictures of the fundus retina. In addition, the input data may include various data. For example, it may include examination data such as eye-related genes and blood.

Preferably, the examination data may include at least one of the pupil size, ATA, ACD, ACW, CLR, and the anterior chamber angle. Here, the anterior chamber angle may be the preoperative anterior chamber angle defined as TIA or SSA. For example, the preoperative anterior chamber angle included in the input data may be TIA750.

The information related to the lens may include a lens size to be applied to the lens implementation. In this case, the lens size may be arbitrarily selected by the user or may be determined through the operation of the same or separate electronic devices.

The configuration of Fig. 8a is exemplary and thus is not intended to limit the scope of the present invention. The apparatus according to the present invention may include some or all the configurations depicted in Fig. 8a, as well as additional or different configurations not shown in Fig. 8a.

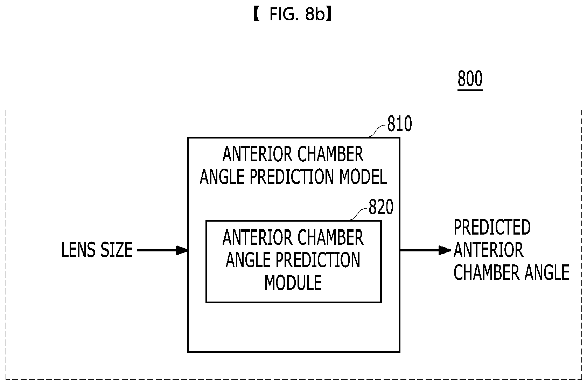

Fig 8b is a diagram illustrating an embodiment of the anterior chamber angle prediction. In view of Fig. 8b, the anterior chamber angle prediction module 810 may predict the postoperative anterior chamber angle by using the lens size of the surgical candidate as the input data.

The anterior chamber angle prediction module 810 may obtain the predicted anterior chamber angle based on the anterior chamber angle prediction model in response to the input data. If the value of the input data changes, the value of the predicted anterior chamber angle may vary. For example, the predicted anterior chamber angle may be output differently depending on the lens size to be inserted into the eye.

According to one embodiment, the anterior chamber angle prediction module 810 may obtain the predicted anterior chamber angle based on the anterior chamber angle prediction model using the lens size. The anterior chamber angle prediction module 810 may output the obtained predicted anterior chamber angle. The predicted anterior chamber angle output from anterior chamber angle prediction module 810 may be output through a display of the electronic device or transmitted to an external device.

According to one embodiment, the input data may further include examination data. A description of the examination data may refer to Fig. 8a. For example, the examination data may further include one of the pupil size, ACD, ACW, CLR, and the preoperative anterior chamber angle. In this case, the anterior chamber angle prediction module 810 may obtain a predicted anterior chamber angle based on the anterior chamber angle prediction model using the lens size and the examination data.

The user can check the predicted anterior chamber angle and determine the appropriate lens size for the user's eye. For example, if the predicted anterior chamber angle meets a predetermined standard (e.g., the anterior chamber angle is 20 degrees or more), the user may determine that the lens size provided as the input data is suitable for the lens implantation.

The anterior chamber angle prediction module 810 may provide the information on whether the input lens size is suitable for the surgical candidate according to the predicted anterior chamber angle. For example, if the predicted anterior chamber angle is less than 20 degrees, it may provide a determination that the lens size is not suitable for the surgical candidate.

According to one embodiment, the predicted anterior chamber angle may be the anterior chamber angle satisfying a predetermined standard. For example, the anterior chamber angle prediction module 810 may output the anterior chamber angle above a predetermined threshold. If the predetermined threshold is 20 degrees and the predicted anterior chamber angle is 35 degrees, the anterior chamber angle prediction module 810 outputs the predicted anterior chamber angle, and the user can determine that the input lens size is suitable for the characteristics of the eye of the surgical candidate.

According to one embodiment, the anterior chamber angle prediction module 810 may output a lens size. For example, if a lens size of 12.6 mm and 30 degrees are output through a display (e.g., the display 220 of Fig. 2), the predicted anterior chamber angle is above the predetermined threshold, and the user can determine that the lens size is suitable for the characteristics of the eye of the surgical candidate. In this case, the output lens size may correspond to the lens size provided as the input data. Alternatively, the anterior chamber angle prediction module 810 may output the lens size together when the predicted anterior chamber angle satisfies a predetermined standard.

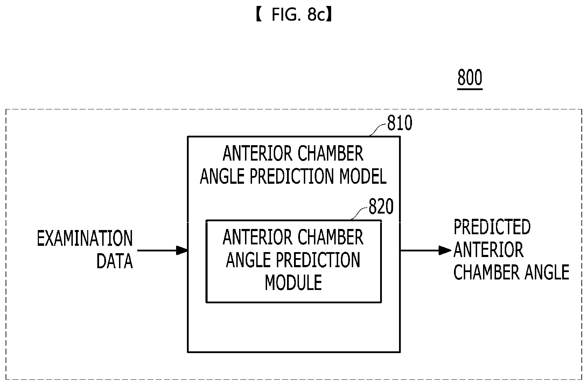

Fig. 8c is a diagram illustrating another embodiment of the anterior chamber angle prediction.

The anterior chamber angle prediction module 810 can predict the lens size and the postoperative anterior chamber angle based on the examination data of the surgical candidate. The anterior chamber angle prediction module 810 may generate a predicted anterior chamber angle and lens size based on the anterior chamber angle prediction model using the examination data. In this case, the lens size may correspond to the predicted anterior chamber angle. The description of the examination data may refer to the description of Fig. 8a.

According to one embodiment, the predicted anterior chamber angle may be the anterior chamber angle satisfying a predetermined standard. The anterior chamber angle prediction module 810 may output the predicted anterior chamber angle satisfying a predetermined standard and output the corresponding lens size. For example, when the predicted anterior chamber angle is 20 degrees or more, the anterior chamber angle prediction module 810 may output the predicted anterior chamber angle and the corresponding lens size.

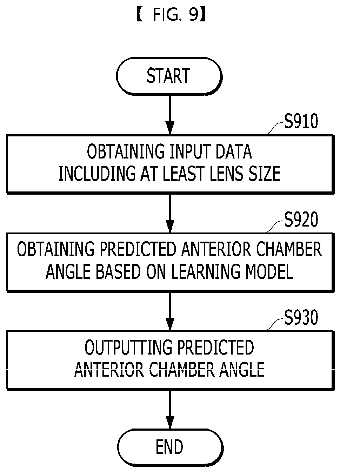

Fig. 9 is a flowchart of the method for predicting the anterior chamber angle according to an embodiment.

In view of Fig. 9, an electronic device (e.g., electronic device 110 of Fig. 1) may obtain the lens size and derive the predicted anterior chamber angle based on the anterior chamber angle prediction model. Although an operation of the electronic device will be described below, the operation of the electronic device can be described as an operation of the anterior chamber angle prediction module (e.g., the anterior chamber angle prediction module 810 of Fig. 8a) or the process (e.g., process 270 of Fig. 2).

In operation S910, the electronic device may obtain the input data including at least a lens size. The lens size may be the lens size may be arbitrarily selected by the user or may be determined through the operation of the same or separate electronic devices.

In operation S920, the electronic device may obtain a predicted anterior chamber angle based on the learning model. The electronic device can obtain the predicted anterior chamber angle based on the learning model using the input data. The electronic device can obtain the predicted anterior chamber angle based on the learning model using at least the lens size. The learning model may include the anterior chamber angle prediction model (e.g., the anterior chamber angle prediction model 820 of Fig. 8A). The anterior chamber angle prediction model may have been trained based on the lens sizes and the anterior chamber angles measured after surgery of a plurality of patients who have undergone the lens implantation in the past.

In operations S910 and S920, the input data may further include examination data. In this case, as being obtained from a plurality of inspection apparatuses, the examination data may include a plurality of examination data. The examination data may be received from an external device (e.g., the second electronic device 120 of Fig. 1), or obtained through a user input, or obtained through an operation of the electronic device. The electronic device can obtain the predicted anterior chamber angle based on the anterior chamber angle prediction model using the examination data and lens size of the surgical candidate.

In operation S930, the electronic device may output the obtained predicted anterior chamber angle. The electronic device may output the predicted anterior chamber angle through a display (e.g., the display 220 of Fig. 2). The electronic device may transmit the obtained predicted anterior chamber angle to the external device. In this case, it is also possible that the electronic device does not directly output the predicted anterior chamber angle.

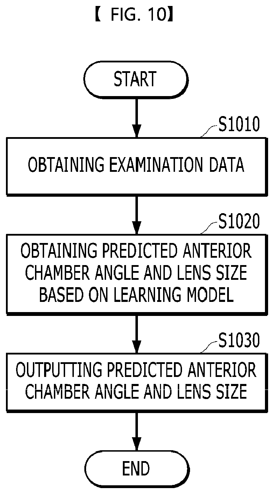

Fig. 10 is a flowchart of the method of predicting the anterior chamber angle according to an embodiment.

In view of Fig. 10, an electronic device (e.g., the electronic device 110 of Fig. 1) may derive the predicted anterior chamber angle and lens size based on the examination data. Although an operation of the electronic device will be described below, the operation of the electronic device can be explained as an operation of the anterior chamber angle prediction module (e.g., the anterior chamber angle prediction module 810 of Fig. 8a) or a process (e.g., the process 270 of Fig. 2).

In operation S1010, the electronic device may obtain that examination data. The description of the examination data may refer to Fig. 8a.

In operation S1020, the electronic device may obtain the predicted anterior chamber angle and lens size based on the learning model. The learning model may include the anterior chamber angle prediction model (e.g., the anterior chamber angle prediction model 820 of Fig. 8a). The electronic device can input the examination data to the learning model and output the predicted anterior chamber angle and lens size based on the anterior chamber angle prediction model.

According to one embodiment, the predicted anterior chamber angle may be the value satisfying a predetermined standard. For example, when the predicted anterior chamber angle is 20 degrees or more, the electronic device may output the predicted anterior chamber angle and lens size.

In operation S1030, the electronic device can output the obtained predicted anterior chamber angle and lens size. In this case, the output lens size may correspond to the output predicted anterior chamber angle.

The method of predicting the anterior chamber angle by the electronic device may not perform all the operations depicted in Fig. 10. The method of predicting the anterior chamber angle may include additional operations, different operations, or fewer operations than those depicted in the figure.

The method of predicting the anterior chamber angle illustrated in in Figs. 9 to 10 is merely exemplary and thus may include additional operations, different operations, or fewer operations than those depicted in the figures.

Although the operations of Figs. 9 to 10 are described as the operation of one electronic device, the operation of predicting the anterior chamber angle may be performed by a plurality of devices. For example, operations S910 to S920 or operations S1010 to S1020 may be performed by a server at a request of the electronic device. The server may transmit the result of operation S920 or operation S1020 to the electronic device. In this case, the electronic device may output the predicted anterior chamber angle and/or lens size obtained from the server.

As such, it can be effective to determine the lens size through the anterior chamber angle prediction for lens implantation. Nevertheless, a bolting value may still be a key factor in determining the lens size. Both bolting values and anterior chamber angle predictions can be used to increase the success rate of the lens implantation.

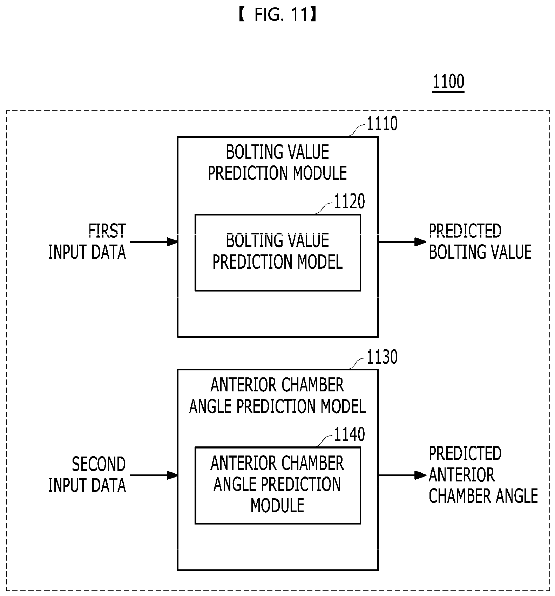

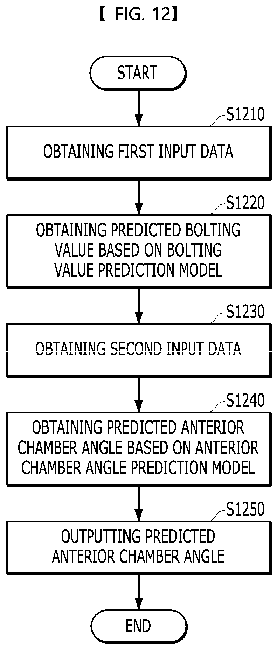

According to one embodiment, the lens size to be input for the anterior chamber angle prediction may be a predetermined lens size value based on the bolting value. Here, the bolting value may include a predicted bolting value determined based on the learning model. For example, the lens size that has been determined by a user after checking the predicted bolting value, or the lens size that has been determined by the electronic device based on the predicted bolting value may be included in the input data of the anterior chamber angle prediction, which will be described in more detail in Figs. 11 and 12.

Fig. 11 illustrates an architecture for the anterior chamber angle prediction according to an embodiment.

According to one embodiment, the architecture 1100 may include a bolting value prediction module 1110 and an anterior chamber angle prediction module 1130. The architecture 1110 may be provided to predict the bolting value based on the first input data and to predict the anterior chamber angle based on the second input data. According to one embodiment, the architecture 800 may be executed by a processor (e.g., processor 270 of Fig. 2).

According to one embodiment, the bolting value prediction module 1110 may include a bolting value prediction model 1120. The bolting value prediction model 1120 may be a learning model configured to detect a predicted bolting value of the eye based on the first input data. The bolting value prediction model 1120 may be a learning model trained based on the examination data of a plurality of patients who have undergone the lens implantation in the past, the lens sizes implanted into the eyes of a plurality of patients, and the bolting values measured after surgery of the plurality of patients.

The first input data may include at least one of the examination data measured by the measuring device or the lens size. The description of the first input data may refer to a description of the input data of the anterior chamber angle prediction module 820 of Figs. 8a-8c.

According to one embodiment, the bolting value prediction model 1120 may, prior to being placed in the electronic device or server, be pretrained on a different electronic device or server or pretrained on the same electronic device or server based on the training data.

The bolting value can be derived differently depending on the lens size to be inserted into the eye. According to one embodiment, the bolting value prediction module 1110 may output the predicted bolting value based on the bolting value prediction model 1120 using the first input data.

According to one embodiment, the predicted bolting value may be the bolting value satisfying a predetermined standard. The bolting value prediction module 1110 may output the predicted bolting value when the predicted bolting value is included within a predetermined range.

For example, if the examination data and the lens size are input as the first input data and the predicted bolting value is included within a predetermined range, the lens size included in the first input data may be suitable for surgery. Alternatively, when the first input data includes the examination data, and the predicted bolting value and the lens size are output, the output lens size may be suitable for surgery if the predicted bolting value is included within a predetermined range.

The user can check the predicted bolting value and primarily determine whether the lens size corresponding to the predicted bolting value is suitable for surgery.

According to one embodiment, the first input data may include a randomly expected lens size to be implanted into the eye of the surgical candidate. The randomly expected lens size may be a standardized or non-standardized lens size.

According to one embodiment, the first input data may include the examination data of the surgical candidate. The input data may include a plurality of parameters. Here, the parameter is defined to indicate the characteristics of the eye of the surgical candidate and can be expressed numerically. For example, the parameters may include at least one of the ATA, ACD-epi, ACD-endo, CCT, CLR, WTW, AL (Axial Length), corneal curvature, flexor abnormalities (myopia, astigmatism, hyperopia), pupil size, IOP, visual acuity, corneal shape, corneal thickness, ocular length, lens insertion space, and the like. In addition, the first input data may include additional data. The description of the first input data may refer to the description of Fig. 8a.

Another description of the bolting value prediction module 1110 and the bolting value prediction model 1120 may refer to the prior art, Korean Application No. 10-2021-002549.

The anterior chamber angle prediction module 1130 may be the same as described in Figs. 8a-8c. Hereinafter, other contents will be mainly explained.

According to an embodiment, the second input data input to the anterior chamber angle prediction module 1130 may be related to the output data of the bolting value prediction module 1110. The second input data may include at least one of the output data of the bolting value prediction module 1110. For example, the second input data input to the anterior angle prediction module 1130 may include the lens size output from the bolting value prediction module 1110.

The lens size may be provided to the anterior chamber angle prediction module 1110 according to the user's selection or input or may be output from the bolting value prediction module 1110 and provided directly to the anterior chamber angle prediction module 1130.

According to one embodiment, the bolting value prediction module 1110 and the anterior angle prediction module 1130 may be connected in series with each other. For example, the predicted bolting value or lens size output from the bolting value prediction module 1110 may be input to the anterior chamber angle prediction module 1130.

The bolting value prediction module 1110 and the anterior chamber angle prediction module 1130 may be implemented by one device and may be implemented by different devices. Alternatively, the bolting value prediction module 1110 and the anterior chamber angle prediction module 1130 may be implemented in conjunction with each other. For example, in order to obtain the lens size to be inserted into the eye of the surgical candidate, the bolting value prediction module 1110 and the anterior chamber angle prediction module 1130 may be implemented in conjunction with each other to derive the predicted bolting value and the predicted anterior chamber angle together with the lens size to be inserted.

As such, the lens size can be derived, and more stable surgical results can be obtained based on the predicted bolting value and the predicted anterior chamber angle. For example, the user can verify the accuracy of the result value derived by the bolting value prediction module 1110. For example, if a lens size of 13.2 mm derived by bolting value prediction module 1110 is input to the anterior chamber angle prediction module 1130 and the derived predicted anterior chamber angle is 30 degrees, it can be verified that the lens size of 13.2 mm is a suitable result value for the eye of the surgical candidate and the accuracy of the result value can also be verified to be high.

The configuration of Fig. 11 is exemplary and thus is not intended to limit the scope of the present invention. The apparatus according to the present invention may include some or all the configurations depicted in Fig. 11, as well as additional or different configurations not shown in Fig. 11.

Fig. 12 is a flowchart of the method of predicting the anterior chamber angle according to an embodiment.

In view of Fig. 12, the electronic device (e.g., the electronic device 110 of Fig. 1) may obtain the input data and derive the predicted anterior chamber angle using the bolting value prediction model and the anterior chamber angle prediction model. Although the operation of the electronic device will be described below, the operation of the electronic device may be explained as the operation of the bolting value prediction module (e.g., the bolting value prediction module 1110 of Fig. 11), the anterior chamber angle prediction module (e.g., anterior chamber angle prediction module 810 of Fig. 8A) or the process (e.g., process 270 of Fig. 2).

In operation S1210, the electronic device may obtain the first input data. The first input data may include at least one of the lens size or the examination data. In this case, as being obtained from a plurality of inspection apparatuses, the examination data may include a plurality of examination data. The lens size may be arbitrarily selected by the user or may be determined based on an additional learning model.

In operation S1220, the electronic device may obtain the predicted bolting value based on the bolting value prediction model using the first input data. The electronic device may obtain the predicted bolting value based on the bolting value prediction model using the first input data.

According to one embodiment, the electronic device may provide a bolting value within a predetermined range as a predicted bolting value. For example, the predetermined range may be from 250 µm to 700 µm.

According to one embodiment, the electronic device may provide a lens size. The electronic device can provide the lens sizes together with the predicted bolting value. Alternatively, if the predicted bolting value satisfies a predetermined range, the electronic device may output the predicted bolting value and the lens size. For example, if the predicted bolting value is 500 µm, the electronic device can output the predicted bolting value and lens size.

Another description of the bolting value prediction module 1110 and the bolting value prediction model 1120 may refer to the prior art, Korean Application No. 10-2021-002549.

In operation S1230, the electronic device may determine the second input data. The second input data may include at least one of the examination data or lens size. In this case, the examination data of operation S 1230 may include parameters of the same type as the examination data included in the first input data but may include parameters of different types.

According to an embodiment, the second input data may be related to the performance result of operation S1220. For example, when the lens size is output together in operation S1220, the second input data may include the lens size.

According to one embodiment, the lens size included in the second input data may be the lens size when the predicted bolting value satisfies a predetermined range. If the predicted bolting value satisfies a predetermined range, the lens size output from the bolting value prediction model may be input to the anterior chamber angle prediction model.

Alternatively, the user may determine whether the corresponding lens size is appropriate based on the predicted bolting value. In this case, in response to the user's input, the electronic device may obtain the lens size. The second input data may include the lens size obtained in response to the user's input.

In operation S1240, the electronic device may obtain the predicted anterior chamber angle based on the anterior chamber angle prediction model using the second input data.

In operation S1250, the electronic device may output the predicted anterior chamber angle. According to one embodiment, the electronic device may output the predicted anterior chamber angle together with the lens size. In this case, the output lens size may correspond to the predicted anterior chamber angle. For example, in Fig. 12, the electronic device may output the lens size included in the second input data in operation S1230.

The electronic device can output both the predicted anterior chamber angle and the predicted bolting value. The user can check the predicted bolting value and the predicted anterior chamber angle to stably determine the lens size to be used for surgery. After performing operation S 1220, the electronic device may first output the predicted bolting value and output the predicted anterior chamber angle in operation S1250 or output the predicted bolting value together with the predicted anterior chamber angle in operation S1250.

The predicted anterior chamber angle may have the value satisfying a predetermined condition. That is, if the electronic device satisfies the predetermined condition, it may output the predicted anterior chamber angle. For example, when the predicted anterior chamber angle is 20 degrees or more, the electronic device may output the predicted anterior chamber angle.

The electronic device may output at least one of the predicted anterior chamber angle, the predicted bolting value, or the lens size through a display (e.g., the display 220 of Fig. 2). The user can determine whether the lens size is appropriate by checking whether the anterior chamber angle is within an appropriate range. Alternatively, although not shown in Fig. 12, the electronic device may transmit at least one of the predicted anterior chamber angle, the predicted bolting value, or the lens size to an external device (e.g., the electronic device 120 of Fig. 1).

The operations of Fig. 12 have been described as the operations of one electronic device, but the described operations may be performed in a plurality of devices. For example, the bolting value prediction in operation S 1220 and the anterior chamber angle prediction in operation S 1240 may be performed on different devices. Alternatively, at least one of operation S1240 and operation S1220 may be performed on the server.

### Model Development

The applicant used statistical and mathematical models to predict the anterior chamber angle after the lens implantation using preoperative data. The measured anterior chamber angles were TIA500, TIA750, SSA500, and SSA750. Using 1) multivariable linear regression (lr), 2) LASSO (Least Absolute Shrinkage and Selection Operator), 3) SVR (Support Vector Machine Regressor) with a Gaussian kernel, 4) Random Forest Regression (RFR), and 5) XGBoost (Extreme Gradient Boosting regression), a predictive model of good performance was derived.

Training data was obtained from 274 patients who have undergone the lens implantation, and the preoperative examination data, the postoperative anterior chamber angle data, and the medical records were used through optical tomography to develop and validate the training dataset. As for the postoperative anterior chamber angle data, the anterior chamber angle data measured after six months since the surgery were considered.

The lens size used a selected value by the user based on an AI-based postoperative bolting value example.

Input variables include the patient's age, gender, SE (spherical equivalent), Km (mean keratometry), pupil size, intraocular pressure (IOP), white-to-white (WHW), central corneal thickness (CCT), angle to angle distance (ATA), anterior chamber depth (ACD), anterior chamber width (ACW), crystalline lens rise (CLR), lens vault (LV), AOD500, AOD750, ARA500, ARA750, TISA500, TISA750, and the lens size.

The variables for the LR model were obtained by running a stepwise method. Variance inflation factors (VIFs) were considered together to avoid multicollinearity. To obtain the interpretation of feature values from the XGBoost model for the anterior chamber angle prediction, the Shapley Additive exPlanations (SHAP) technique was used.

The mean squared error (MAE) and Pearson's correlation coefficients between the anterior chamber angle obtained after surgery and the predicted anterior chamber angle were used to evaluate the regression model. The data were analyzed using a linear mixed-effect model to derive more accurate results. In addition, the postoperative TIA and SSA regression model results using the ROC (receiver operating characteristic) curve were evaluated and areas under the curves (AUC) were calculated to evaluate the predictive performance of the developed model.

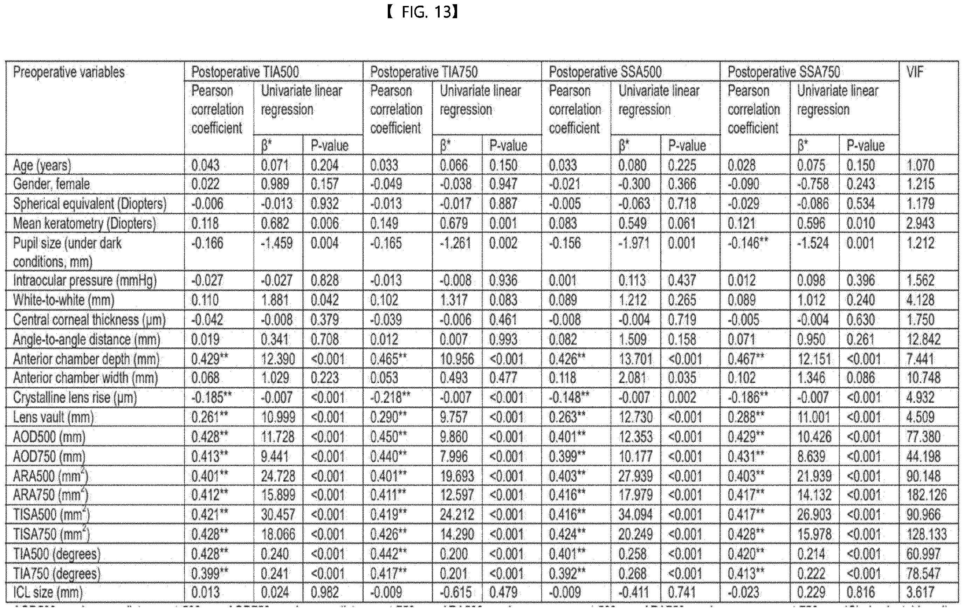

Fig. 13 illustrates the relationship between the preoperative eye measurement data and the postoperative anterior chamber angle. Specifically, it shows the correlation coefficient for the relationship between the preoperative eye measurement value and the postoperative anterior chamber angle using the training dataset, univariate regression analysis results, and VIF.

In view of Fig. 13, the preoperative variables ACD, CLR, LV, AOD, ARA, TISA, and TIA exhibit a significant correlation (P < 0.001) with postoperative TIAs and SSAs. Some of these variables, such as ATA or ACW, exhibit very high VIF values, which can cause multicollinearity problems. In developing the following predictive model, variables with high VIF may not be considered.

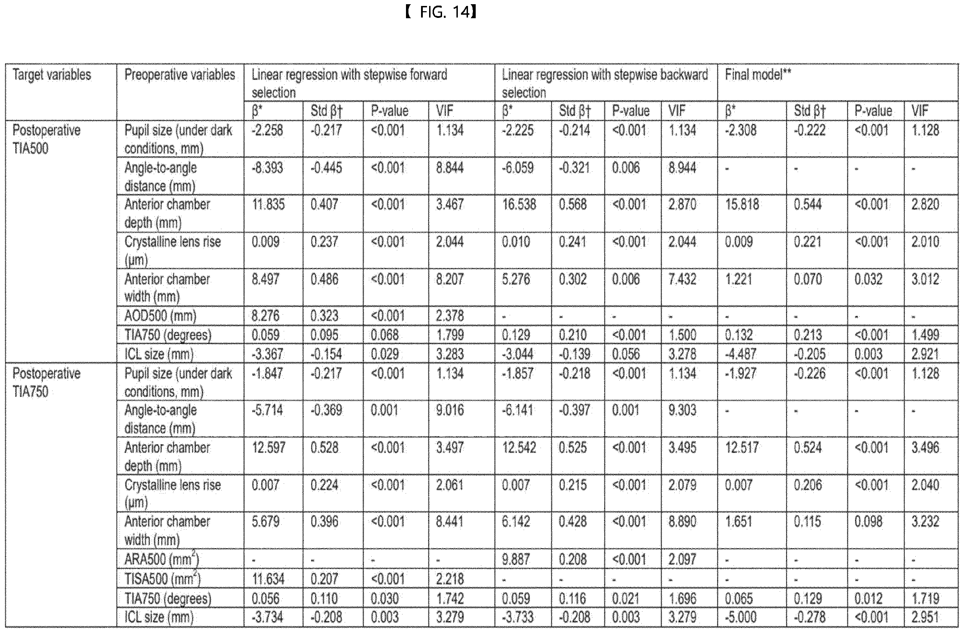

Fig. 14 shows the results of the multivariate LR analysis for a postoperative TIA. According to the LR model of stepwise selection, the pupil size, ATA, ACD, ACW, CLR, AOD500, and TIA 750 were shown to be the important input variables for postoperative TIA 500. However, AOD 500 was removed because it failed to be selected in both phased selections. ATA was also removed due to the high VIF. Finally, the pupil size, ACD, ACW, CLR, TIA 750 and lens size were chosen for the final model to predict TIA 500. The same variables were chosen for the TIA 750 through the same process.

The lens size and the predicted postoperative anterior chamber angle are negatively correlated with each other. This means that a large lens size can lead to a narrow anterior chamber angle. In addition, the data distribution and multivariate regression analysis results indicate that the inserted lens size interacts with the ACD in predicting the postoperative anterior chamber angle.

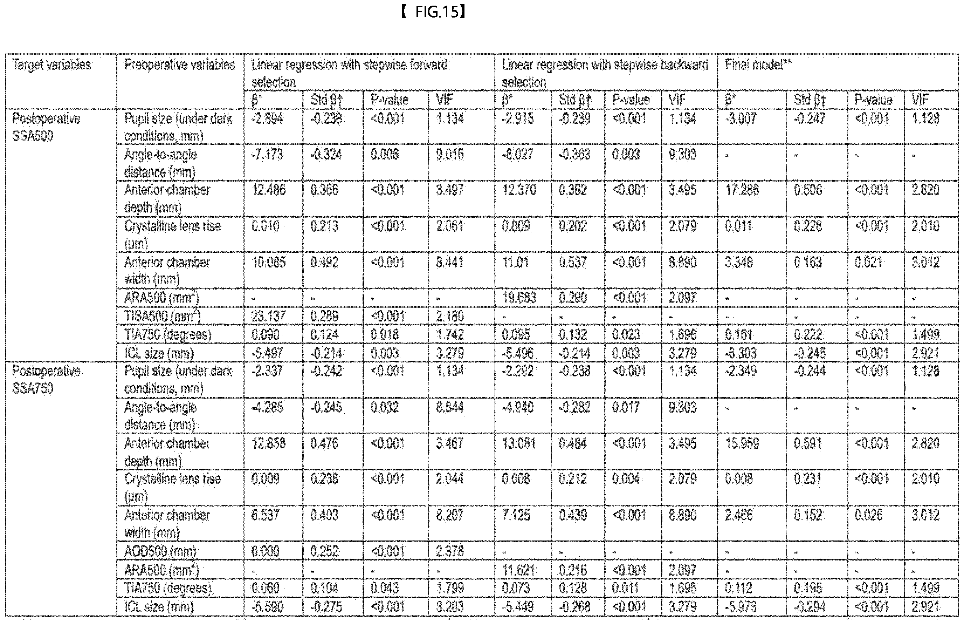

Fig. 15 depicts the results of the multivariate LR analysis for the postoperative SSA. Through the stepwise selection and removal of the variable due to a high VIF, pupil size, ACD, ACW, CLR, TIA 750 and ICL sizes were chosen as the final input variables for the final LR model for the postoperative SSA.

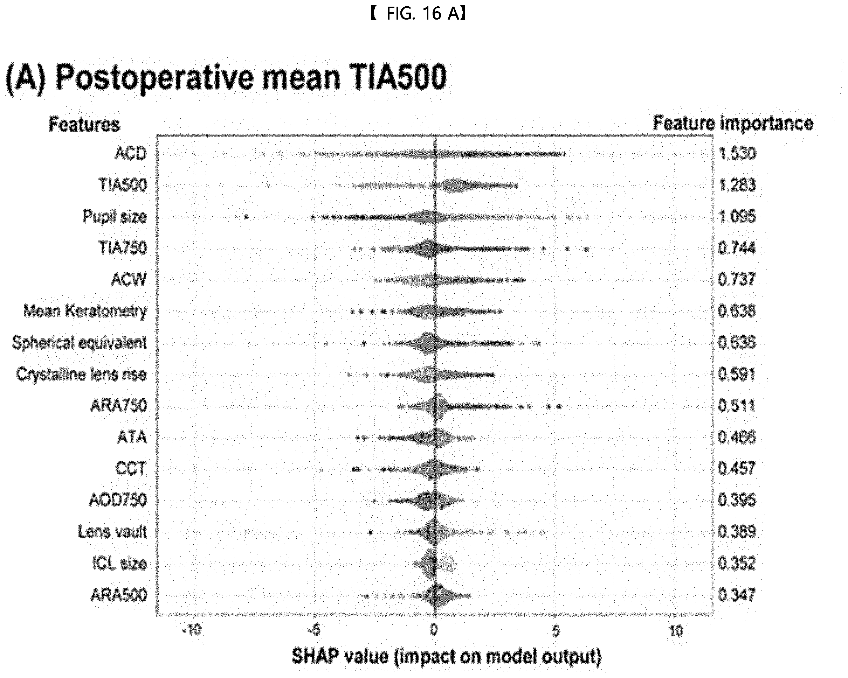

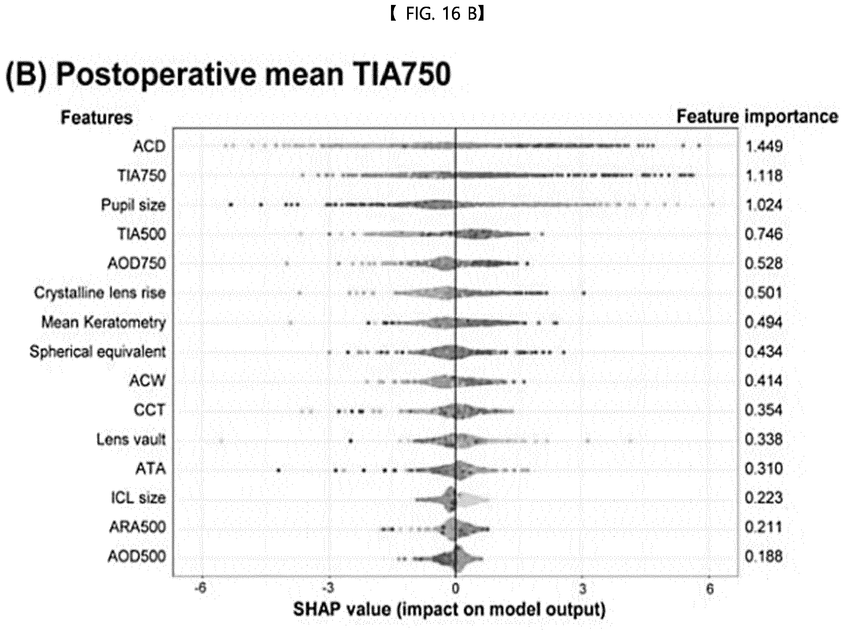

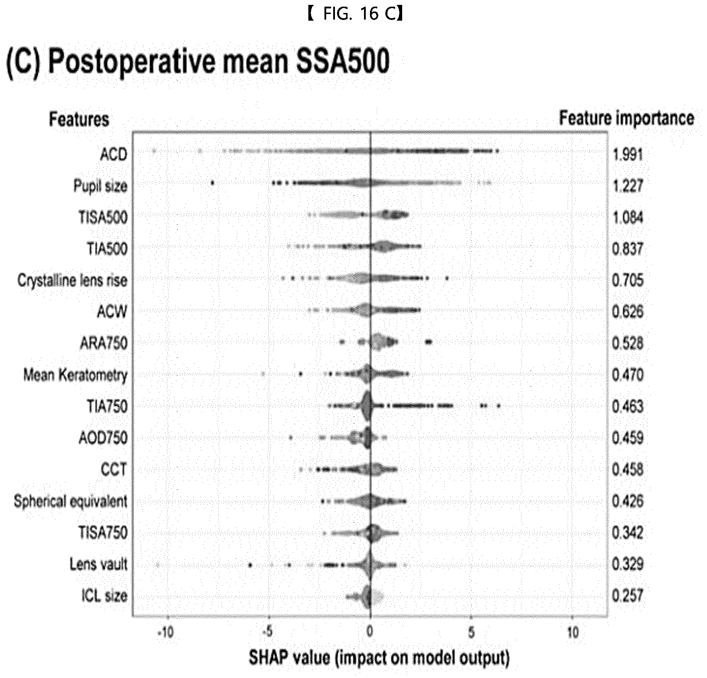

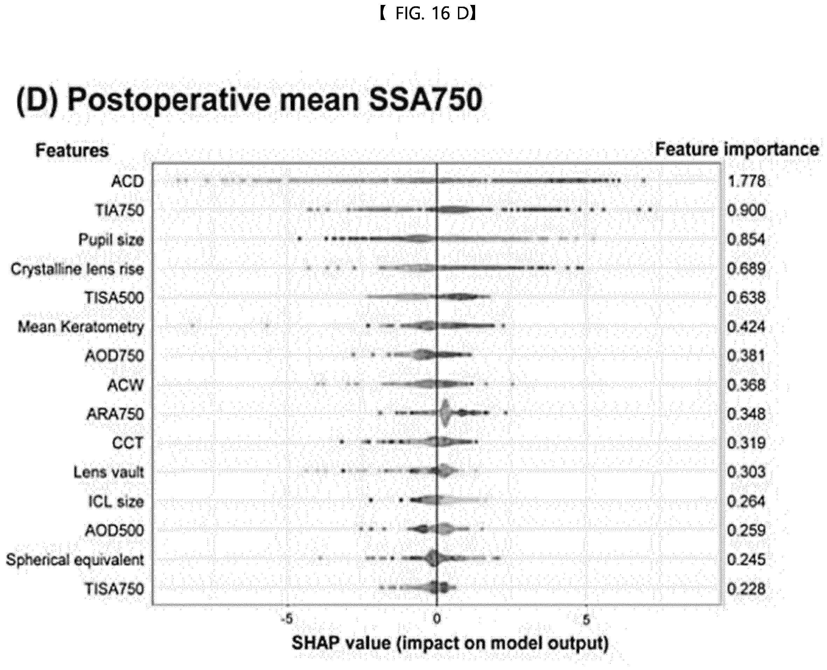

Fig. 16 depicts the Shapley value of each predictor for the anterior chamber angle prediction model according to various embodiments of the present invention. Specifically, it shows the relationship between the SHAP value and each characteristic. In particular, it demonstrates the Shapley plot in the XGBoost model.

According to SHAP, ACD appears to be the most important predictor for the anterior chamber angle prediction. In view of Fig. 16, the top five predictors of the anterior chamber angle prediction model for TIA 500 are ACD, TIA500, pupil size, TIA 750, and ACW. For TIA 750, the top five predictors of the anterior chamber angle prediction model are ACD, TIA 750, pupil size, TIA 500, and AOD 750. For SSA 500, the top five predictors of the anterior chamber angle prediction model are ACD, pupil size, TISA500, TIA500, and CLR. For the SSA750, the top five predictors of the anterior chamber angle prediction model are ACD, TIA750, pupil size, CLR, and TISA 500.

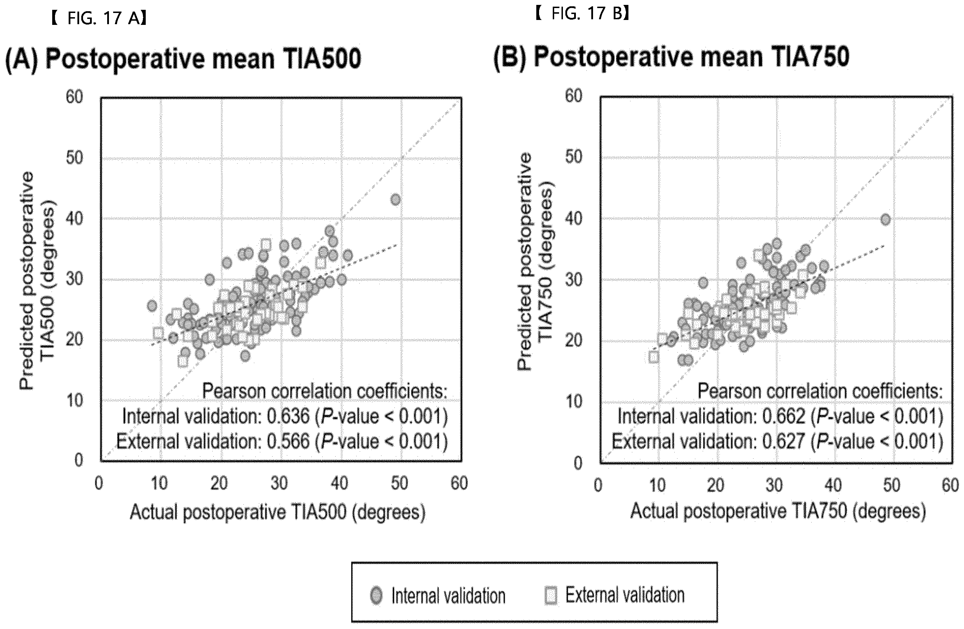

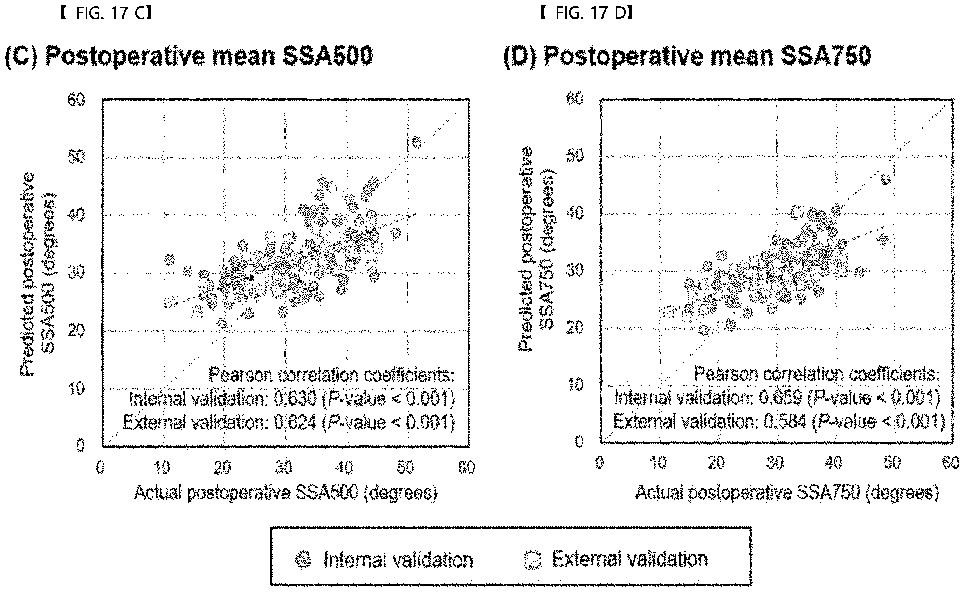

Fig. 17 depicts the results of comparing the predicted postoperative anterior chamber angle with the actual anterior chamber angle according to various embodiments of the present invention.

As a result of calculating the Pearson correlation coefficients between the actual and predicted anterior chamber angles for the internal validation dataset, the Pearson coefficient was 0.636 for the TIA500, 0.662 for the TIA 750, 0.630 for the SSA500 and 0.0659 for the SSA750.

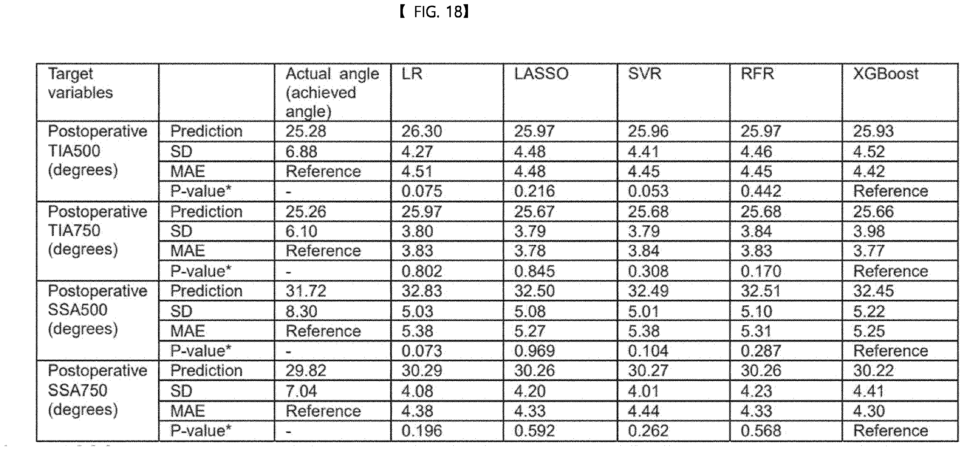

Fig. 18 depicts the results of comparing the predicted postoperative anterior chamber angle with the actual anterior chamber angle according to various embodiments of the present invention.

Using the anterior chamber angle prediction model, the predicted anterior angle was obtained on the internal validation dataset and compared with the actual anterior angle. In view of Fig. 18, the lowest minimum average error (MAE) value was found in the XGBoost model as a result of deriving the MAE between the actual anterior chamber angle and the predicted anterior chamber angle. Nevertheless, the differences were negligible when compared to other models.

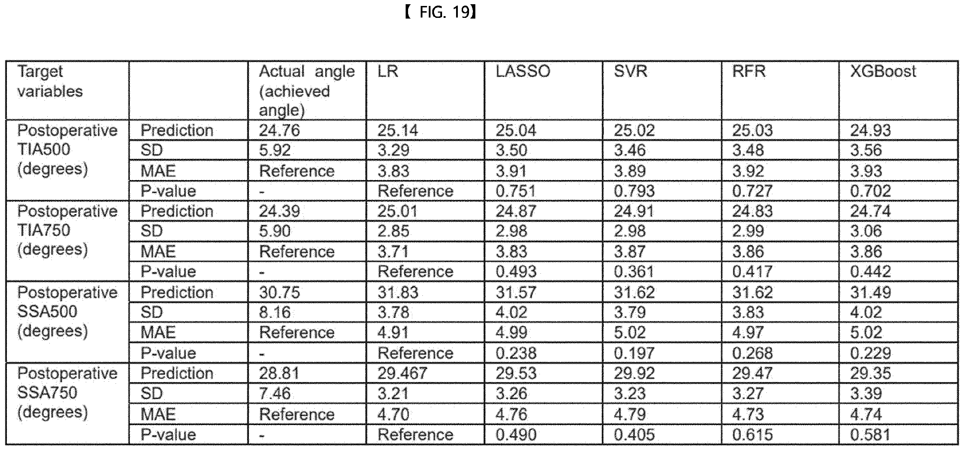

Fig. 19 depicts the results of comparing the actual postoperative anterior chamber angle and the predicted anterior chamber angle according to various embodiments of the present invention.

The anterior chamber angle prediction model according to various embodiments of the present invention was used to obtain the predicted anterior chamber angle on an external verification dataset to be compared with the actual anterior chamber angle. In view of Fig. 19, the lowest MAE value was found in the LR model as a result of deriving the MAEs between the actual anterior chamber angle and the predicted anterior chamber angle. Nevertheless, the differences were negligible when compared to other models.

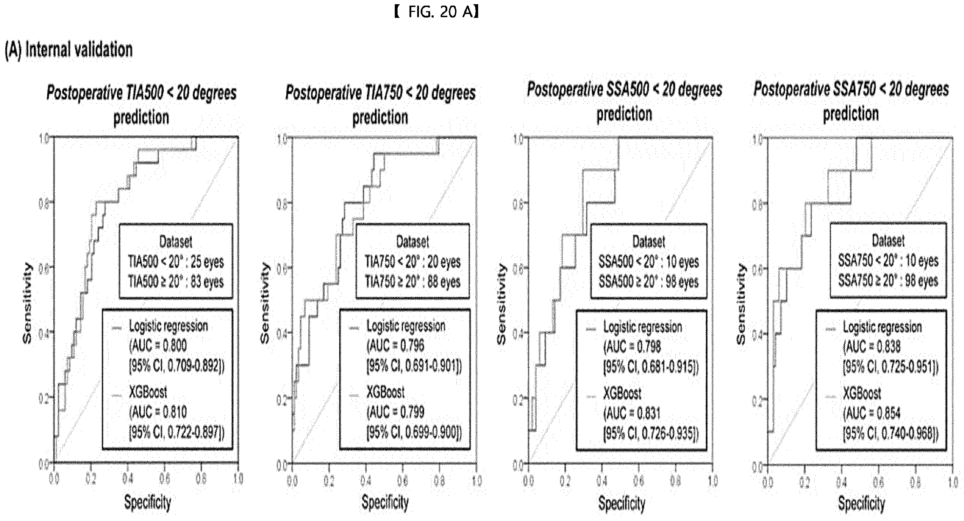

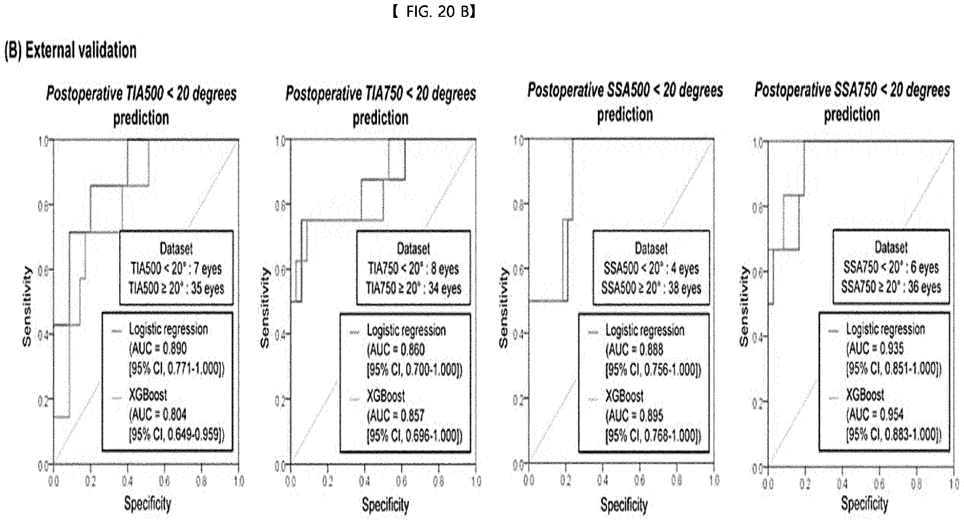

Fig. 20 depicts the ROC AUC of the anterior chamber angle prediction model according to various embodiments of the present invention.

The developed anterior chamber angle prediction model was used to detect a narrow postoperative anterior chamber angle (< 20°). The models developed here used the LR model and the XGBoost model.

In view of Fig. 20, the AUC for all the prediction result, the external verification dataset, and the internal validation dataset exhibited a value of 0.79 or more. In addition, due to the small number of the narrow anterior chamber angle data, 5-fold cross-validation was performed, and similar results were obtained with the 5-fold cross-validation.

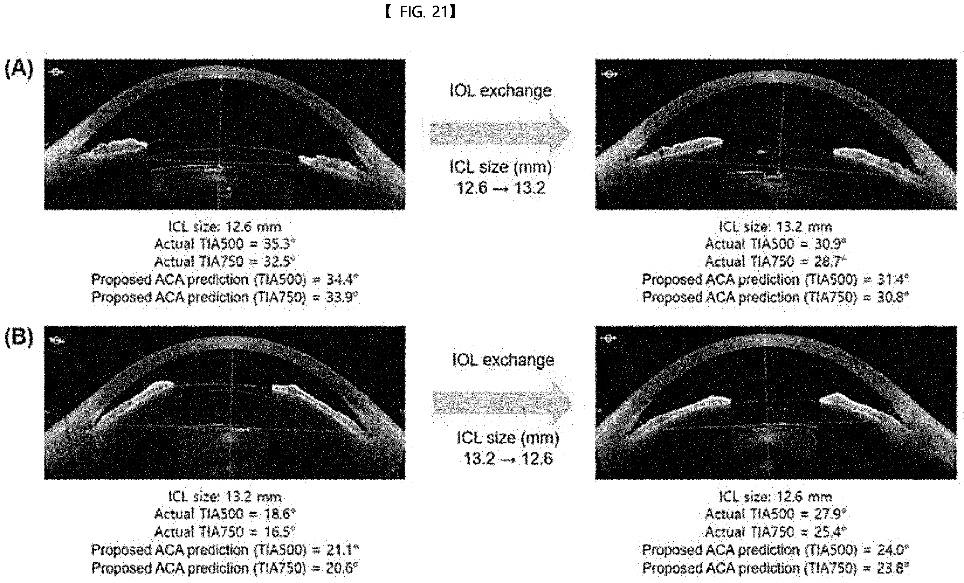

Fig. 21 depicts a change in the anterior chamber angle according to a change in the lens size.

In the case of changing the lens size, the actual postoperative anterior chamber angle was measured, and the postoperative anterior chamber angle was obtained based on the anterior chamber angle prediction model according to various embodiments of the present invention.

In view of (a) in Fig. 21, in the case of enlarging the lens size, both the actual postoperative anterior chamber angle and the predicted postoperative anterior chamber angle decreased. Conversely, in view of (b) in Fig. 21, in the case of reducing the lens size, both the actual postoperative anterior chamber angle and the predicted postoperative anterior chamber angle increased.

Some experiments have confirmed that the size of the inserted lens size can lead to the narrow predicted postoperative anterior chamber angle. If the appropriate lens size is selected, the phenomenon of the narrow anterior chamber angle caused by factors such as the pupil size, ACD, ACW, CLR, and preoperative anterior chamber angle can be alleviated.

In addition, the bolting value was identified as a factor almost independent from the narrow anterior chamber angle. However, the bolting value may still be an important factor in determining the lens size. Therefore, considering both the bolting value and the anterior chamber angle according to the embodiments of the present invention will help to derive a successful surgical result in consideration of the correct lens value.

In addition, according to the anterior chamber angle prediction model proposed in the present invention, it will be feasible to predict the postoperative anterior chamber angle without considering age, SE, AL, etc.

In addition, in terms of the data characteristics, a technique for predicting the anterior chamber angle using one or more models of LR, LASSO or ML may be appropriate.

Although the foregoing description illustrates the various components of the present invention operating in combination, it is important to note that the invention is not necessarily limited to these embodiments. The scope of the invention allows for the selective combination and operation of its components, either as a whole or in part insofar as such is within the range aimed by the present invention.

Meanwhile, various embodiments described herein may be implemented by a hardware, middleware, microcode, software, and/or a combination thereof. For example, various embodiments may be implemented within one or more application-specific semiconductors (ASICs), digital signal processors (DSPs), digital signal processing devices (DSPDs), programmable logic devices (PLDs), and field programmable gate arrays (FPGAs), processors, controllers, microcontrollers, other electronic units designed to perform the functions presented herein, or combinations thereof.

In addition, for example, various embodiments may be included or encoded in a computer-readable medium containing instructions. Instructions included or encoded in a computer-readable medium may cause a programmable processor or other processor to execute the method, for example, when instructions are executed. The computer-readable medium includes a computer storage medium, and the computer storage medium may be any available medium that can be accessed by a computer. For example, such a computer-readable medium may include RAM, ROM, EEPROM, CD-ROM or other optical disc storage media, a magnetic disk storage media, or other magnetic storage devices.

Such a hardware, software, firmware, etc. may be implemented within the same device or individual devices to support the various operations and functions described herein. Additionally, the components, units, modules, components, etc. described in the present invention as "... unit" may be implemented together or separately as individual but interoperable logic devices. The description of different features for modules, units, etc. is intended to emphasize different functional embodiments and does not necessarily mean that they must be realized by individual hardware or software components. Rather, functions associated with one or more modules or units may be performed by individual hardware or software components or integrated into common or individual hardware or software components.

The operations are depicted in the drawings in a specific order, but it should not be understood that these operations are to be performed in the specific order, or sequential order, or that all illustrated operations need to be performed to accomplish the desired result. In some environments, multitasking and parallel processing may be advantageous. Moreover, the separation of the various components in the embodiments set forth in the foregoing should not be understood as requiring such a separation in all embodiments, and it must be understood that the described components may generally be integrated together into a single software product or packaged into multiple software products.

An electronic device, server, or external device according to the various embodiments of this document described above may include at least one of, for example, a smartphone, a tablet PC, a mobile phone, a video telephone, a desktop PC, a laptop PC, a personal digital assistant (PDA), a portable multimedia player (PMP), a MP3 player, a mobile medical device, a camera, or a wearable device.

According to the various embodiments, the wearable device may be at least one of an accessory type (e.g., a watch, ring, bracelet, anklet, necklace, eyeglasses, contact lenses, or head-mounted-device (HMD)), textile or apparel all-in-one (e.g., electronic clothing), a body attachment type (e.g., a skin pad or tattoo), or a bioimplantable type (e.g., an implantable circuit).

In some embodiments, the electronic device or external device may be a home appliance. The home appliances may include, for example, at least one of a television, a DVD player (Digital Video Disk players), an audio, a refrigerator, an air conditioner, a vacuum cleaner, an oven, a microwave oven, a washing machine, an air purifier, a set-top box, a home automation control panel, a security control panel, a TV box, a game console, an electronic dictionary, an electronic key, a camcorder, or an electronic picture frame.

In another embodiment, the electronic device, the external device, and the wearable device may be used in various medical apparatuses (e.g., various portable medical measuring devices, such as blood glucose meters, heart rate monitors, blood pressure monitors, or temperature monitors, etc.), magnetic resonance angiography (MRA), magnetic resonance imaging (MRI), computed tomography (CT), cinematograph, or ultrasound), navigation devices, global navigation satellite systems (GNSS), event data recorder (EDR), flight data recorder (FDR), automotive infotainment devices, domestic robots, or Internet of Things devices (e.g., light bulbs, various sorts of sensors, electric or gas meters, sprinkler devices, smoke alarms, thermostats, street lights, fitness equipment, hot water tank, heater, boiler, etc.).

As above, the optimal embodiments have been demonstrated in the drawings and specifications. Certain terms used herein are used only for the purpose of describing the invention but not for limiting the meaning or scope of the invention described in the claims. Therefore, those skilled in the art will appreciate that various variations and other equivalent embodiments are possible therefrom. Therefore, the true scope of technical protection of the present invention should be determined by the technical spirit of the appended claims.

## Claims

1. A method of predicting the anterior chamber angle of the eye of a surgical candidate for lens implantation, the method comprising:
obtaining input data including a lens size; and
obtaining a predicted postoperative anterior chamber angle based on a learning model using the input data.
wherein the learning model is based on the lens size and the measured postoperative anterior chamber angle of a plurality of patients who have undergone the lens implantation in the past.

2. The method of predicting the anterior chamber angle according to claim 1, the method further comprising outputting the obtained lens size.

3. The method of predicting the anterior chamber angle according to claim 1, wherein the obtained lens size is determined based on a bolting value.

4. The method of predicting the anterior chamber angle according to claim 1, wherein the input data further comprises examination data of the surgical candidate.

5. The method of predicting the anterior chamber angle according to claim 1, wherein the examination data comprises at least one of pupil size, anterior chamber depth (ACD), anterior chamber width (ACW), crystalline lens rise (CLR), or preoperative anterior chamber angle.

6. The method of predicting the anterior chamber angle according to claim 1, wherein the anterior chamber angle is defined as a trabecular iris angle (TIA) or a scleral spur angle (SSA).

7. An apparatus for predicting the anterior chamber angle after surgery, the apparatus comprising:
a memory for storing a learning model trained based on the lens size and the measured postoperative anterior chamber angle of a plurality of patients who have undergone the lens implantation in the past; and
at least one processor electrically connected to the memory,
wherein the processor, when executed, obtains input data including a lens size, and obtains a predicted postoperative anterior chamber angle based on a learning model using the input data.
